(19) Europäisches Patentamt
European Patent Office
Office européen des brevets

(11) Publication number : **0 341 735 B1**

(12) # EUROPEAN PATENT SPECIFICATION

(45) Date of publication of patent specification : **30.09.92 Bulletin 92/40**

(51) Int. Cl.$^5$ : **A61K 31/70**

(21) Application number : **89108596.1**

(22) Date of filing : **12.05.89**

(54) **N-acetyl-3-fluoroneuraminic acid as antiviral drug.**

(30) Priority : **13.05.88 JP 116500/88**

(43) Date of publication of application : **15.11.89 Bulletin 89/46**

(45) Publication of the grant of the patent : **30.09.92 Bulletin 92/40**

(84) Designated Contracting States : **AT BE CH DE FR GB IT LI LU NL SE**

(56) References cited :
**EP-A- 0 281 067**
**AGRIC. BIOL. CHEM., vol. 52, no. 5, May 1988; T.NAKAJIMA et al., pp. 1209-1215***
**CARBOHYDRATE RESEARCH, vol. 127, 1984, Elsevier Science Publishers BV, Amsterdam (NL); M.N.SHARMA et al., pp. 201-210***
**E. WIESMANN, "Medizinische Mikrobiologie", 1978, Georg Thieme Verlag, Stuttgart (DE); J.ECKERT et al., pp. 316-323***

(73) Proprietor : **MECT CORPORATION**
**2-1-1, Nishi-Shinjyuku Shinjyuku-ku**
**Tokyo (JP)**

(72) Inventor : **Suda, Isao**
**2886-5, Yayoi-cho**
**Tokorozawa-shi Saitama-ken (JP)**
Inventor : **Ohrui, Hiroshi**
**5-2-901, Odawarayamamoto-cho**
**Sendai-shi Miyagi-ken (JP)**
Inventor : **Meguro, Hiroshi**
**1-14-3, Tsurugaya**
**Sendai-shi Miyagi-ken (JP)**
Inventor : **Ido, Tatsuo**
**Kawauchi Jutaku 3-104, Mubanchi Kawauchi**
**Sendai-shi Miyagi-ken (JP)**

(74) Representative : **Vossius & Partner**
**Siebertstrasse 4 P.O. Box 86 07 67**
**W-8000 München 86 (DE)**

**EP 0 341 735 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an antiviral drug.

It is known that a sialic acid is positioned at an end of a glycochain of a glycoprotein or glycolipid and is greatly related to the physiological activity of the sugar chain. for elucidating the function of the sialic acid, the production of sialic acid derivatives is thought important.

The fluorinated derivatives of N-acetyl neuraminic acid having a fluorine atom in 2-position (M. N. Shana and R. Eby, Carbohydr. Res., Vol 127, p. 201, 1984) or in 9-position (M. Sharma and W. Korytnyl, J. Carbohydr. Chem., Vol. 1, p. 311, 1982 to 1983) were already produced. N-acetyl-3-fluoroneuraminic acid derivatives and a method for their preparation are diclosed in EP-A-281 067 (published after the priority date of the present invention).

It has now been found that N-acetyl-3-fluoroneuraminic acid remarkably inhibits the activity of sialidase which plays an important role in the viral or bacterial infection.

It is the object of the present invention to provide an antiviral drug comprising N-acetyl-3-fluorneuraminic acid or a pharmacologically acceptable salt thereof.

The present invention relates to an antiviral drug comprising N-acetyl-3-fluoroneuraminic acid of the following formula:

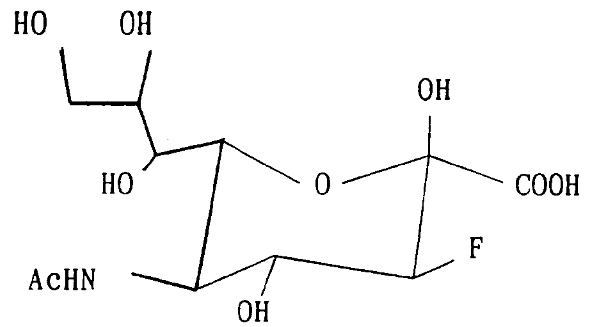

( I )

or a pharmacologically acceptable salt thereof, which drug is in the form of a preparation for injection, inhalation or oral administration.

Fig. 1 is a Dixon plot showing an inhibition pattern of N-acetyl-3-fluoroneuraminic acid.

N-acetyl-3-fluoroneuraminic acid of the above formula (I) is produced by, for example, as follows: either (1) an inert gas containing fluorine or (2) acetyl hypofluorite produced by introducing an inert gas containing fluorine into a mixture of acetic acid and an acetic acid salt is introduced into an aqueous acetic acid solution or dichloromethane solution of an alkyl (e.g. $-CH_3$) 5-acetamido-2, 6-anhydro-4,7,8,9-tetra-0-acetyl-2,3,5-trideoxy-D-glycero-D-galacto-2-enonate of the following formula (II):

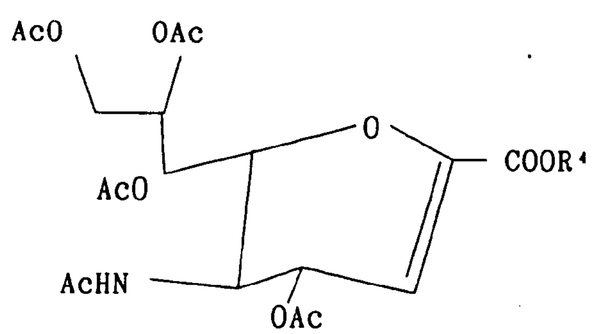

(II)

wherein $R^4$ represents a methyl group and Ac represents an acetyl group, to conduct the addition reaction.

The compound of the above general formula II is producible by, for example, a process disclosed in "Dai-9-kai T shitsu Symposium K en Yoshish (the 9th Glucide Symposium) p. B11."

As the inert gas containing fluorine, argon gas containing 1 to 2% of fluorine is used. The gas is introduced into the solution of the compound of the above general formula II in an aqueous acetic solution or in dichloromethane at a low temperature of around 7°C for 1 to 2 hr to form a difluorinated product having a fluorine atom

at 2-position and 3-position in cis form as well as two kinds of monofluorinated products having $-OCOCH_3$ at 2-position and a fluorine atom at 3-position in cis-and trans-form. When acetyl hypofluorite is used, the monofluorinated product is mainly formed.

The monofluorinated product is separated from the reaction liquid and purified by distilling off the solvent under reduced pressure, subjecting the residue to a column chromatography with silica gel, eluting the product with, for example, an ethyl acetate/ether mixture and recrystallizing it.

The monofluorinated product thus formed is deacetylated to form N-acetyl-3-fluoroneuraminic ester, and its ester group is then removed to produce the above-described N-acetyl-3-fluoroneuraminic acid.

N-acetyl-3-fluoroneuraminic acid is effective for various viral infections. This fact is proved by its effect of inhibiting the action of sialidase (neuraminidase) [see "Tanpakushitsu Kakusan K so (Protein, Nucleic Acid and Enzyme)," Vol. 25, No. 1 (1980), pages 30 to 48].

The effective dose of N-acetyl-3-fluoroneuraminic acid is usually 1 μg to 10 mg.

N-acetyl-3-fluoroneuraminic acid is usable in various forms. It is administered by injection, inhalation or oral administration.

In order to prepare the antiviral drug of the present invention, various kinds of additives can be incorporated into the drug. Such additives include a diluent, a binder, a disintegrator, a lubricant or a film forming agent.

The diluent includes glucose, starch, lactose, mannitol, sorbitol, sucrose, kaolin, dextrin, cyclodextrin, titanium oxide, anhydrous calcium phosphate, light anhydrous silicate, talc, natural hydrated magnesium silicate, precipitated calcium carbonate, magnesium metasilicate aluminate, crystalline cellulose, calcium carboxymethyl cellulose, and mixtures thereof.

The binder includes starch, dextrin, Traganth gum, gelatin, polyvinylpyrrolidone, polyvinyl alcohol, methyl cellulose, ethyl cellulose, hydroxyethyl cellulose, hydroxypropyl cellulose, hydroxypropylmethyl cellulose, hydroxypropyl starch, crystalline cellulose, pectine, polypectine, sodium polypectinate, polyhydroxyethyl methacrylate, carboxymethyl cellulose, sodium carboxymethyl cellulose, Acacia gum, sodium alginate, and mixtures thereof.

The disintegrator includes starch, crystalline cellulose, carboxymethyl cellulose, calcium carboxymethyl cellulose, carboxymethyl starch, hydroxypropyl starch, powdered agar, mannan, and mixtures thereof.

The lubricant includes talc, stearic acid, magnesium and calcium stearate, hydrogenated oil, sesame oil, paraffin, and mixtures thereof.

The film forming agent includes cellulose derivatives such as ethyl cellulose (EC), carboxymethyl cellulose (CMEC), cellulose acetate (CA), cellulose acetate phthalate (CAP), hydroxypropylmethyl cellulose (HPMC), methyl cellulose (MC), hydroxypropyl cellulose (HPC); and polyvinyl derivatives such as polyvinyl acetal diethylamino acetate, methacrylic acid/ ethyl acrylate copolymer, methacrylic acid/ methyl acrylate copolymer, ethyl methacrylate/ methacrylic acid trimethyl ammonium chloride ethyl copolymer and dimethylaminoethyl methacrylate/ methyl acrylate copolymer.

When the film forming agent is coated on the drug, a conventional coating aid such as a plasticizer as well as other various additives for preventing the medicines from adhering to each other when the coating is made, are added to the drug of the present invention, in order to improve the property of the film forming agent and to make easier the coating procedure.

Further, the following additives can be used for preparing the present drug in the form of inhalation or injection.

Calcium chloride, potassium chloride, sodium chloride, hydrochloric acid, citric acid, sodium citrate, succinic acid, acetic acid, potassium acetate, sodium acetate, tartaric acid, potassium hydroxide, sodium hydroxide, injection water, sodium chloride injection, sodium carbonate, sodium bicarbonate, lactic acid, sodium lactate, maleic acid, sulfuric acid, phosphoric acid, potassium phosphate, sodium phosphate, potassium hydrogen phosphate, calcium hydrogen phosphate, potassium dihydrogen phosphate, sodium hydrogen phosphate, sodium dihydrogen phosphate, sodium phosphite, sodium dihydrogen phosphite, potassium pyrophosphite, sodium pyrophoshite, ℓ-arginine, purified egg yolk lecithin, purified gelatin, gelatin, lactose, human serum albumin, polyethylene glycol 400, 1,500, 4,000 and 6,000, polyoxyethylene hydrogenated castor oil 60, and sodium ethylenediamine tetraacetate.

The following Reference Examples and Examples will further illustrate the present invention.

Reference Example 1

Argon gas containing 1.7% (6.1 mmol) of fluorine ($F_2$) was introduced, for 2 hr, into 25 ml (7°C) of an acetic acid solution of 2.3 g (4.8 mmol) of methyl 5-acetamido-2,6-anhydro-4,7,8,9-tetra-O-acetyl-2,3,5-trideoxy-D-glycero-D-galacto-2-enonate of the following formula IIa:

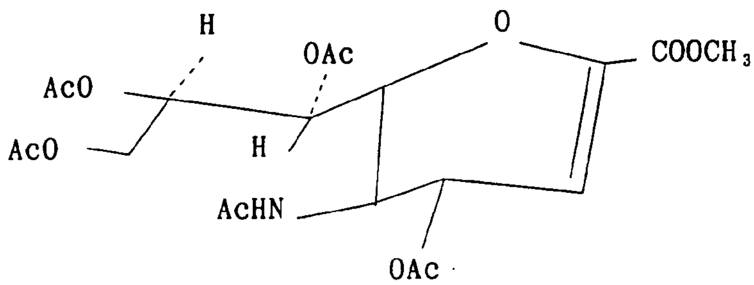

(IIa)

wherein Ac represents an acetyl group (the same shall apply hereinafter).

Then, the solvent was distilled off under reduced pressure, to obtain 2.5 g of a syrup. The syrup was treated according to a column chromatography with silica gel (250 g of G 60; a product of Merck Co.), followed by the elution with a solvent mixture of ethyl acetate and ether (volume ratio: 3/1) and recrystallization from a solvent mixture of dichloromethane and isopropyl ether to form the following three products: (1) Methyl 5-acetamido-4,7,8,9-tetra-0-acetyl-,2,3,5-trideoxy-2,3-difluoro-D-erythro-β-L-gluco-2-nonulosonate:

[Molecular structure]

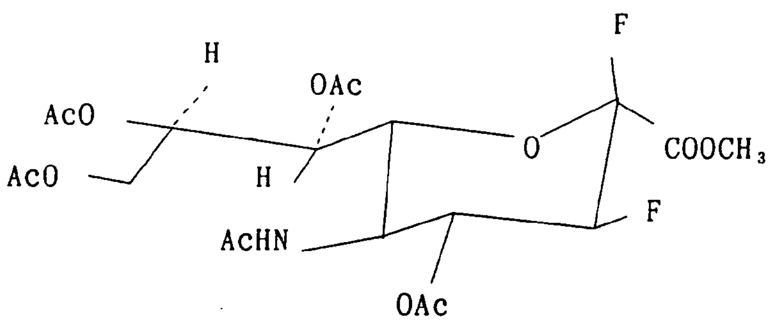

Yield: 900 mg (36%)

[Physical properties]

Melting point: 92°C

$[\alpha]^{20}_{D}$ - 48.12° (C=0.55, chloroform)

Elementary analysis: $C_{20}H_{27}N_1O_{11}F_2$

| | |
|---|---|
| Calculated: | C:46.97; H:5.32; N:2.73 |
| Found: | C:47.00; H:5.37; N:3.00 |
| | Rf=0.54 (ethyl acetate) |

$^1$H-NMR δ ($CDCl_3$)

5.00(H-3, $J_{3,4}$ = 9.85 Hz)
5.39(H-4, $J_{4,5}$ =10.26 Hz)
4.40(H-5, $J_{5,6}$ =10.66 Hz)
4.27(H-6, $J_{6,7}$ = 2.38 Hz)
5,39(H-7, $J_{7,8}$ = 8.06 Hz)
5.16(H-8, $J_{8,9}$ = 5.68 Hz, $J_{8,9'}$ = 2.71 Hz)
4.01, 4.32(H-9, H-9', $J_{9,9'}$ = 12.55 Hz)
5.49(NH, $J_{NH,5}$ = 10.20 Hz)
3.91(COO$\underline{CH_3}$)
1.91(NCO$\underline{CH_3}$)
2.05, 2.10, 2.11, 2.13(OAc)

(2) Methyl 5-acetamido-2,4,7,8,9-penta-O-acetyl-3,5-dideoxy-3-fluoro-D-erythro-β-L-gluco-2-nonulosonate:[Molecular structure]

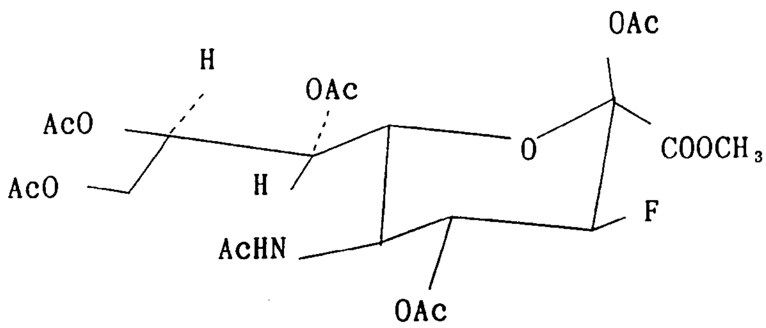

Yield: 190 mg (7%)

[Physical properties]

Melting point: 119°C

$[\alpha]_D^{20}$ - 56.5° (C=0.21, $CHCl_3$)

Elementary analysis: $C_{22}H_{30}NO_{14}F$

| | |
|---|---|
| Calculated: | C:47.91; H:5.48; N:2.53 |
| Found: | C:48.10; H:5.35; N:2.30 |
| | Rf=0.26 |

$^1$H-NMR δ ($CDCl_3$)

4.62(H-3, $J_{3,4}$ = 9.60 Hz)
5. 48(H-4, $J_{4,5}$ =10. 05 Hz)
4. 24(H-5, $J_{5,6}$ =10. 54 Hz)
4.11(H-6, $J_{6,7}$ - 2.29 Hz)
5,35(H-7, $J_{7,8}$ = 4.66 Hz)
5.02(H-8, $J_{8,9}$ = 6.69 Hz, $J_{8,9'}$ = 2.44 Hz)
4.14 4.51(H-9, H-9', $J_{9,9'}$ = 12.46 Hz)
5.58(NH, $J_{NH,5}$ = 10.08 Hz)
3.85($COOCH_3$)
1.89($NCOCH_3$)
2.04, 2.06, 2.10, 2.15, 2.24(OAC)

(3) Methyl 5-acetamido-2,4,7,8,9-penta-O-acetyl-3,5-dideoxy-3-fluoro-D-erythro-β-L-manno-2-nonulosonate:[Molecular structure]

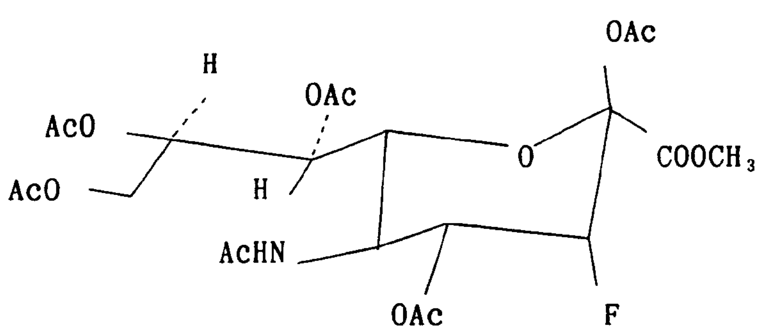

Yield: 30 mg, (7%)

[Physical properties]

Melting point: 194°C
$[\alpha]_D^{20}$ - 24.5° (C=0.19, chloroform)
Elementary analysis: $C_{22}H_{30}NO_{14}F$

| | |
|---|---|
| Calculated: | C:47.91; H:5.48; N:2.53 |
| Found: | C:47.85; H:5.53; N:2.55 |
| | Rf=0.23 (ethylacetate) |

$^1$H-NMR δ ($CDCl_3$)
4.94(H-3, $J_{3,4}$ = 2.57 Hz)
5.50(H-4, $J_{4,5}$ =10.68 Hz)
4.14(H-5, $J_{5,6}$ =10.61 Hz)
4.25(H-6, $J_{6,7}$ = 1.84 Hz)
5.35(H-7, $J_{7,8}$ = 5.13 Hz)
5.13(H-8, $J_{8,9}$ = 6.60 Hz, $J_{8,9'}$ = 2.20 Hz)
4.57(H-9, $J_{9,9'}$ = 12.46 Hz)
5.43(NH, $J_{NH,5}$ = 8.79 Hz)
3.84(COO$\underline{CH_3}$ )
1.92(NCO$\underline{CH_3}$ )
2.04, 2.05, 2.11, 2.17, 2.18(OAc)

Reference Example 2

Argon gas containing 1.5% (10 mmol) of fluorine ($F_2$ ) was introduced into a cartridge containing a mixture of potassium acetate and acetic acid, to form acetyl hypofluorite (AcOF). AcOF was introduced into 10 ml of an acetic acid solution of 480 mg (1.0 mmol) of methyl 5-acetamido-2,6-anhydro-4,7,8,9-tetra-O-acetyl-2,3,5-trideoxy-D-glycero-D-galacto-2-enonate (the same solution as that used in Example 1) at room temperature for 1 hr. Then, the solvent was distilled off at 60°C under reduced pressure, to obtain 520 mg of a syrup.

The syrup was treated according to the column chromatography with silica gel (60 g of G 60; a product of Merck Co.), followed by the elution with a solvent mixture of ethyl acetate and ether (volume ratio: 3/1) and recrystallization from a solvent mixture of dichloromethane and isopropyl ether to form the same products as those formed in Reference Example 1 as follows:

(1) Methyl 5-acetamido-4,7,8,9-tetra-O-acetyl-2,3,5-trideoxy-2,3-difluoro-D-erythro-β-L-gluco-2-nonulosonate
[yield: 40 mg (7.7%)], and
(2) Methyl 5-acetamido-2,4,7,8,9-penta-O-acetyl-3,5-dideoxy-3-fluoro-D-erythro-β-L-gluco-2-nonulosonate
[yield: 190 mg (34%)].

Reference Example 3

1 ml of methanol containing 2 mmol of sodium methoxide ($NaOCH_3$ ) was added to 3 ml of a solution of 55 mg of methyl 5-acetamido-2,4,7,8,9-penta-O-acetyl-3,5-dideoxy-3-fluoro-D-erythro-β-L-gluco-2-nonulosonate [(2) produced in Reference Example 1 or 2] in anhydrous methanol,and the mixture was stirred at room temperature for 30 min. A slightly excess amount of Dowex 50 [$H^+$ ] was added to the reaction liquid and the mixture was stirred. The resin was removed by filtration, and then washed with 5 ml of methanol. The filtrate was concentrated under reduced pressure and recrystallized from a solvent mixture of methanol, ethyl acetate and petroleum ether, to obtain the following compound (6) [yield: 24 mg (75%)]. (6) Methyl 5-acetamido-3,5-dideoxy-3-fluoro-D-erythro-β-L-gluco-2-nonulosonate

[Molecular structure]

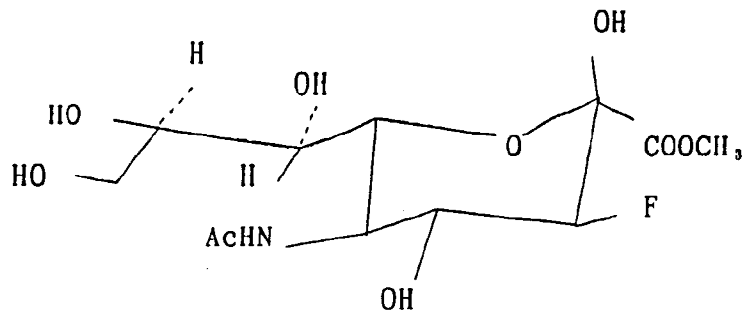

[Physical properties]

Melting point: 209°C (recrystallized from a solvent mixture of methanol, ethyl acetate and petroleum ether)

$[\alpha]_D^{20}$ - 41.14° (C=0.86, $H_2O$)

Elementary analysis: $C_{12}H_{20}NO_9F$

Calculated: C:42.23; H:5.9; N:4.1

Found: C:42.37; H:6.0; N:4.3

$^1$H-NMR δ ($D_2O$)

4.75(H-3, $J_{3,4}$ = 8.98 Hz)

4.09-4.18(3H, H-4, H-5, H-6)

3.56(H-7, $J_{6,7}$ = 1.10 Hz)

3.71(H-8, $J_{7,8}$ = 8.79 Hz

$J_{8,9}$ = 6.05 Hz,

$J_{8,9}$ = 3.43 Hz)

3.63, 3.8(H-9, H-9', $J_{9,9'}$ =11.9 Hz)

Reference Example 4

20 mg (0.036 mmol) of methyl 5-acetamido-2,4,7,8,9-penta-O-acetyl-3,5-dideoxy-3-fluoro-D-erythro-β-L-gluco-2-nonulosonate produced in Reference Example 1 or 2 was added to 1ml of a methanol solution containing 20% of a 1 N aqueous NaOH solution, and the resultant mixture was stirred at room temperature for 1 h.

Then, 1 ml of a methanol solution containing 2 mmol of sodium methoxide ($NaOCH_3$) was added to the reaction liquid and the mixture was stirred at room temperature for 30 min. A slightly excess amount of Dowex 50 [$H^+$] was added to the reaction liquid and the mixture was stirred. After filtration, the resin was washed with 10 ml of water. The filtrate was concentrated under reduced pressure. The residue was dissolved in water. A small amount of active carbon was added to the solution and the mixture was stirred. Then, the active carbon was filtered off and the filtrate was dried in vacuum, to obtain the following compound (7).
[yield: 9 mg (76 %)].
(7) 5-Acetamido-3,5-dideoxy-3-fluoro-D-erythro-β-L-gluco-2-nonulosonic acid

[Molecular structure]

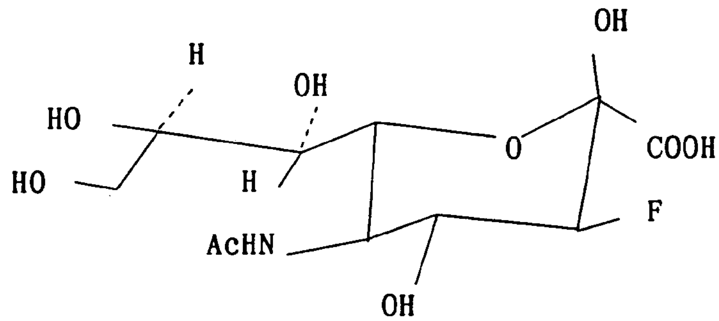

[Physical properties]

Melting point: 205°C
$[\alpha]_D^{20}$ - 40.02° (C=0.86, $H_2O$)
Elementary analysis: $C_{11}H_{18}NO_9F$
Calculated: C:40.37; H:5.54; N:4.27
Found: C:40.07; H:5.51; N:4.21
$^1$H-NMR δ ($D_2O$)
4.71(H-3, $J_{3,4}$ = 8.42 Hz)
4.10-4.15(3H, H-4, H-5, H-6)
3.54(H-7, $J_{6,7}$ = 0 Hz)
3.73(H-8, $J_{7,8}$ = 8.79 Hz
$J_{8,9}$ = 6.05 Hz),
$J_{8,9}$ = 2.75 Hz)
3.63, 3.83(H-9, H-9′, $J_{9,9'}$ = 11.91 Hz)
2.06($NCOCH_3$)

Example 1

100 μℓ of a 0.5 mM solution of 4-methylumbelliferyl-N-acetylneuraminic acid (4-MU-NeuAc) (a product of Nakarai Kagaku Co., Ltd) of the following formula:

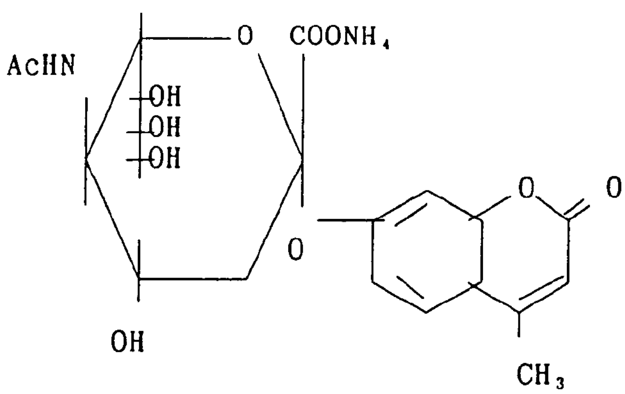

,

100 μℓ of a 200 mM sodium acetate buffer (pH 4.2) and 100 μℓ of N-acetyl-3-fluoroneuraminic acid (compound 1) 4 to 400 μM were added to an enzyme solution (100 mℓ) (10 mU/ml) of sialidase (a product of Nakarai Kagaku Co., Ltd.) product from Arthrobacter ureafaciens in a test tube, and the reaction was conducted in a water bath at 37 °C for 15 min. Then, 3.6 ml of a 200 mM glycine/NaOH buffer (pH 10.4) was added to the reaction mixture in the test tube to terminate the reaction. the concentration of 4-methylumbelliferone formed in the reaction

solution was determined from its fluorescence intensity at an excitation wave length of 360 nm and an emission wave length of 440 nm with a fluorescence spectrophotometer (a product of Hitachi, Ltd.). The amount of free sialic acid was determined from this value.

The sialidase inhibition rate was determined according to the following formula:

$$\text{Inhibition rate (\%)} = \left(1 - \frac{\text{Amount of free sialic acid in the presence of compound 1}}{\text{Amount of free sialic acid in the absence of compound 1}}\right) \times 100$$

The results are shown in Table 1 and Fig. 1.

It is apparent from Table 1 that Compound 1 remarkably inhibits the activity of sialidase.

Fig. 1 is a Dixon plot showing the reaction rates obtained by using two different substrate concentrations in the presence of Compound 1. It is apparent from Fig. 1 that the inhibition of Compound 1 is competitive with the substrate.

Table 1

Inhibiting effect on sialidase produced by Arthrobacter **ureafaciens:**

| Compound 1 (mM) | NeuAc released (n mol) | Inhibition rate (%) |
|---|---|---|
| None | 27.3 | |
| 0.1 | 4.9 | 82.0 |
| 0.01 | 14.0 | 48.9 |
| 0.001 | 23.0 | 14.3 |

Examples 2 and 3

The same procedure as that of Example 1 was repeated except that sialidases produced by Vibrio colerae and Streptococcus sp. were used, and then the inhibition rate of Compound 1 was determined. The results are shown in the following Tables 2 and 3.

Table 2

Inhibiting effect on sialidase produced by Vibrio colerae:

| Compound 1 (mM) | NeuAc released (n mol) | Inhibition rate (%) |
|---|---|---|
| None | 5.8 | |
| 0.1 | 2.8 | 52.7 |
| 0.01 | 5.3 | 8.4 |
| 0.001 | 5.9 | -0.2 |

Table 3

Inhibiting effect on sialidase produced by Streptococcus sp.

| Compound 1 (mM) | NeuAc released (n mol) | Inhibition rate (%) |
|---|---|---|
| None | 11.3 | |
| 0.1 | 2.8 | 75.4 |
| 0.01 | 8.5 | 24.6 |
| 0.001 | 10.5 | 7.4 |

It is apparent from the above tables that Compound 1 had an inhibiting effect on sialidases produced by various microorganisms.

(Acute toxicity)

The acute toxicity test of Compound 1 was conducted by an intravenous injection in female mice.

Experiment conditions:

Animals used: Female ICR mice (6 week old)
Medicine concentration: 13 to 15 W/V % in distilled water
Number of mice for each level: 10
Observation period: 14 days.

Results:

The results are shown in Table 4. It is apparent that the toxicity of Compound 1 was quite low.

Table 4

| Administration method | Dose (mg) | Number of deaths |
|---|---|---|
| Intravenous injection | 200 | 0 |
| | 400 | 0 |
| | 600 | 0 |

## Claims

1. An antiviral drug comprising N-acetyl-3-fluoroncuraminic acid of the following formula:

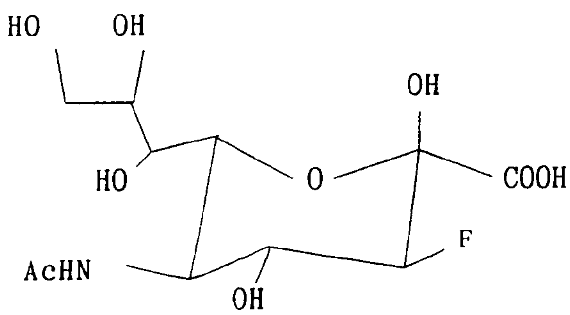

(I)

or a pharmacologically accepatble salt thereof, which drug is in the form of a preparation for injection, inhalation or oral administration.

2. The antiviral drug of claim 1, wherein said N-acetyl-3-fluoroneuraminic acid is contained in an amount of 1 µg to mg.

3. N-acetyl-3-fluoroneuraminic acid of the formula I for use as an antiviral agent in the form a preparation for injection, inhalation or oral administration.

## Patentansprüche

1. Antivirales Arzneimittel, das N-Acetyl-3-fluoroneuraminsäure der folgenden Formel umfaßt,

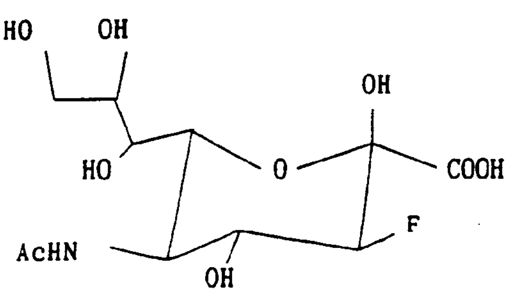

(I)

oder ein pharmazeutisch verträgliches Salz davon, wobei das Arzneimittel in Form eines Präparats zur Injektion, Inhalation oder oralen Verabreichung vorliegt.

2. Antivirales Arzneimittel nach Anspruch 1, wobei die N-Acetyl-3-fluoroneuraminsäure in einer Menge von 1 µg bis 10 mg enthalten ist.

3. N-Acetyl-3-fluoroneuraminsäure der Formel I zur Verwendung als antivirales Arzneimittel in Form eines Präparats zur Injektion, Inhalation oder oralen Verabreichung.

## Revendications

1. Médicament antiviral comprenant de l'acide N-acétyl-3-fluoroneuraminique de la formule suivante :

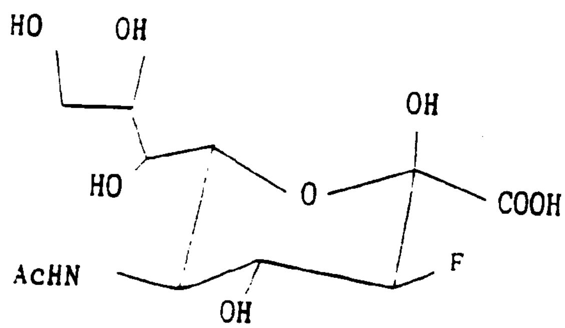

(I)

ou un sel pharmacologiquement acceptable de celui-ci, lequel médicament est sous la-forme d'une préparation pour injection, inhalation ou administration orale.

2. Médicament antiviral suivant la revendication 1, caractérisé en ce que l'acide N-acétyl-3-fluoroneuraminique est contenu en une quantité de 1 µg à 10 mg.

3. Acide N-acétyl-3-fluoroneuraminique de la formule I, utilisable comme agent antiviral, sous la forme d'une préparation pour injection, inhalation ou administration orale.

FIG.1

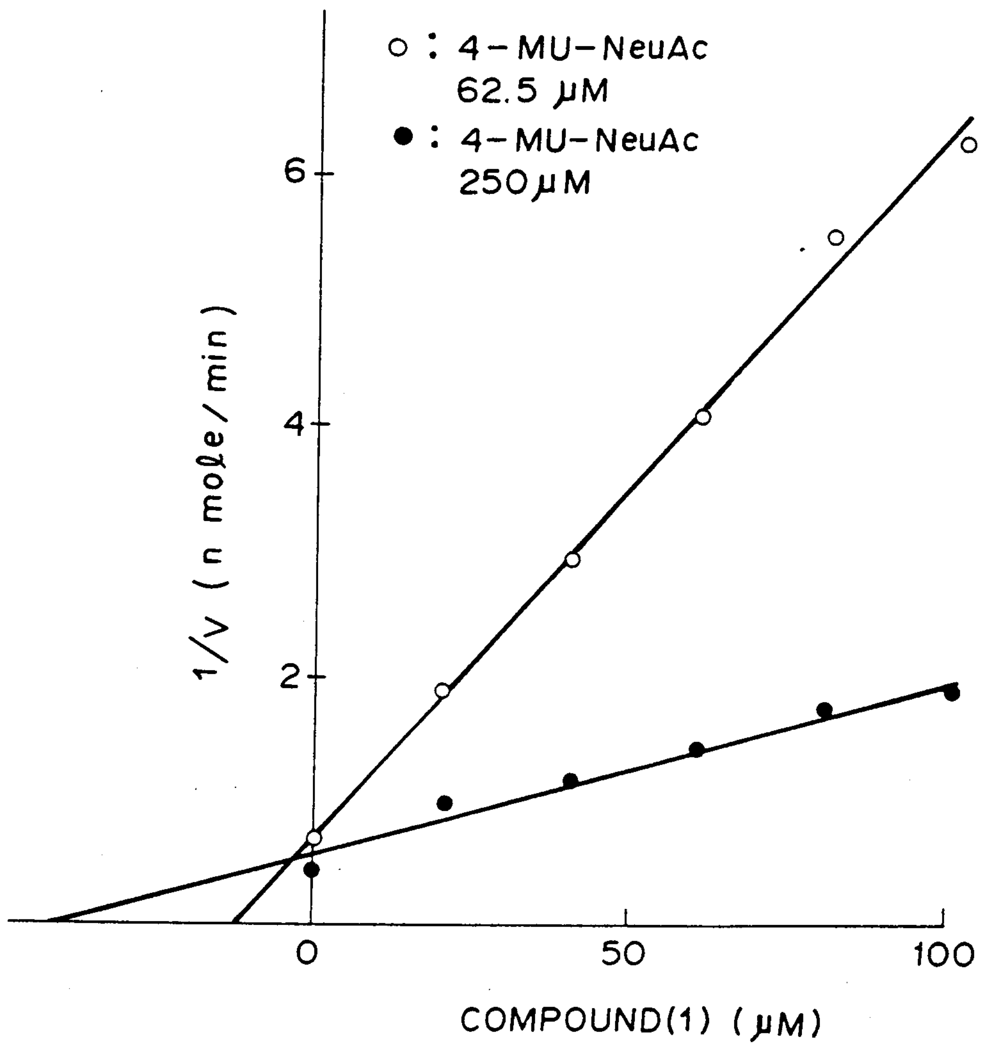
○ : 4-MU-NeuAc
62.5 μM
● : 4-MU-NeuAc
250μM
1/V (n mole/min)
6
4
2
0
50
100
COMPOUND(1) (μM)